# EUROPEAN PATENT SPECIFICATION

(11) **EP 1 940 249 B1**
(45) Date of publication and mention of the grant of the patent: **03.06.2015**
(21) Application number: 06806421.1
(22) Date of filing: 20.10.2006
(51) Int. Cl.: A23L 1/30, A23L 1/29, A23L 1/303, A23L 1/275, A23K 1/16, A61K 8/00

(54) **NOVEL FORMULATIONS OF FAT-SOLUBLE ACTIVE INGREDIENTS WITH HIGH BIOAVAILABILITY**
NEUE FORMULIERUNGEN VON FETTLÖSLICHEN AKTIVEN INHALTSSTOFFEN MIT HOHER BIOVERFÜGBARKEIT
NOUVELLES FORMULATIONS D'INGRÉDIENTS ACTIFS LIPOSOLUBLES AYANT UNE BIODISPONIBILITÉ ÉLEVÉE

(30) Priority: 21.10.2005 EP 05023060; 14.11.2005 EP 05024813
(43) Date of publication of application: 09.07.2008
(73) Proprietor: DSM IP Assets B.V., 6411 TE Heerlen (NL)
(72) Inventor: BECK, Markus, 79540 Lörrach (DE); GRASS, Hansjoerg, 4132 Muttenz (CH); LEUENBERGER, Bruno, H., CH-4123 Allschwil (CH); NOWOTNY, Markus, CH-4132 Muttenz (CH); SCHAEFER, Christian, 79618 Rheinfelden (DE); VOELKER, Karl, Manfred, 79111 Freiburg (DE)
(74) Representative: Steck, Melanie
(86) International application number: PCT/EP2006/010120
(87) International publication number: WO 2007/045488

(56) References cited:
- EP-A- 1 405 572
- WO-A-03/015537
- WO-A2-03/022071
- US-A1- 2002 110 599
- US-A1- 2003 232 892

## Description

The present invention relates to novel formulations of a fat-soluble active ingredient with high bioavailability of said fat-soluble active ingredient as well as to their manufacture and use as dietary supplement, food, feed, personal care product and/or Pharmaceutical.

There is a need to provide formulations of fat-soluble active ingredients with high bioavailability of said fat-soluble active ingredient which reduces the amount of fat-soluble active ingredients needed to be incorporated by animals including humans.

This need is fulfilled by the formulation of the present invention which comprises a composition of a fat-soluble active ingredient and a protective colloid characterized in that said composition when dissolved, dispersed or diluted in/with water has an extinction E1/1 at a wavelength in the range of from 200 to 800 nm, preferably at a wavelength in the range of from 250 to 600 nm, more preferably at a wavelength in the range of from 250 to 500 nm, more preferably at a wavelength in the range of from 370 to 485 nm, of ≥ 380, preferably of ≥ 600, most preferably of ≥ 900. Such compositions are also called "colour-intensive compositions" in the context of the present invention.

In preferred embodiments of the formulations of the present invention the formulation also shows an extrusion loss of fat-soluble active ingredient of ≤ 30%, preferably of ≤ 15%, more preferably of ≤ 10%, most preferably of ≤ 5% when pressed to tablets, i.e. the amount of fat-soluble active ingredient present at the surface of tablets of these formulations is ≤ 30%, preferably ≤ 15 weight-%, more preferably ≤ 10 weight-%, most preferably ≤ 5 weight-%, based on the total weight of the fat-soluble active ingredient in the formulation. Fat-soluble active ingredient present at the surface of such a tablet is a great disadvantage since the fat-soluble active ingredient is no longer protected against oxidation by the protective colloid.

The extrusion loss is determined by
o cautious milling of the tablets to a mix so that the formulation itself is not destroyed by using a mortar;
o treating said mix with a suitable solvent (e.g. methylene chloride or petrolether) so that only the fat-soluble active ingredient which has been pressed out is dissolved;
o diluting the solution (solvent + fat-soluble active ingredient) with another solvent -(cyclohexane or isopropanol) and
o analytical determination of the fat-soluble active ingredient in the solvent by measuring the absorption of the solution, and
o calculation of the percentage of the total amount of the fat-soluble active ingredient pressed out.

The extrusion loss is a relevant parameter for the shelf life of (pharmaceutical) tablets, i.e. a parameter for the stability of the fat-soluble active ingredient in the (pharmaceutical) tablets. If the extrusion loss is smaller, the shelf life of the tablets is longer.

Further advantages of the formulations of the present invention are their easy handling properties, their good content uniformity and the low dust formation during handling.

Thus, the present invention is also directed to a process for the determination of a formulation of a fat-soluble active ingredient with high bioavailability comprising the following steps:
i) providing a sample of the formulation containing a composition of the fat-soluble active ingredient and the protective colloid;
ii) preparing a dispersion of said formulation in water;
iii) measuring the extinction coefficient E1/1 of water and of said solution at the wavelength in the range of from 200 to 800 nm,
iv) subtracting the extinction coefficient E1/1 for water from the one of said solution.

If the extinction coefficient E1/1 is at a wavelength in the range of from 200 to 800 nm ≥ 380 the formulation is one with high bioavailability.

The fat-soluble ingredients are those with a pharmacological effect or those providing health benefits to the human or animal body in general. Preferably they are selected from the group consisting of carotenoids and the fat-soluble vitamins (vitamin A, vitamin E, vitamin K, vitamin D, coenzyme Q10, polyunsaturated fatty acids such as eicosapentaeneoic acid and docosahexaeneoic acid and their triglyceride esters), as well as their physiologically acceptable derivatives such as their esters, especially with C₁₋₂₀ carbonic acids, and any mixtures of them.

Preferred examples of carotenoids are β-carotene, α-carotene, astaxantin, lutein, zeaxanthin, cryptoxanthin, 8'-apo-β-carotenal, 8-apo-β-carotenoic acid esters such as the ethyl ester, canthaxanthin, lycopene, crocetin, α- or β-zeacarotene and citranaxanthin, as well as their physiologically acceptable derivatives such as their esters, especially with C₁₋₂₀ carbonic acids, and any mixtures of them.

More preferably the fat-soluble active ingredient is selected from the group consisting of β-carotene, 8'-apo-β-carotenal, lutein, zeaxanthin, lycopene, astaxantin, canthaxanthin, citranaxanthin, 8'-apo-β-carotenoic acid ethyl ester and any mixture of them.

Most preferred carotenoids are β-carotene, lutein, lycopene, astaxantin, canthaxanthin, and zeaxanthin, especially β-carotene, lutein, lycopene, and zeaxanthin.

The term "β-carotene" encompasses the all-cis as well as the all-trans isomers and all possible mixed cis-trans-isomers. The same applies for the other carotenoids.

The term "zeaxanthin" encompasses the natural R,R-zeaxanthin, as well as S,S-zeaxanthin, meso-zeaxanthin and any mixture of them. The same applies for lutein.

The fat-soluble active ingredients may be of natural origin, i.e. isolated/extracted from plants, purified and/or concentrated, as well as those synthesized by chemical and/or microbiological (fermentative) routes.

The formulation of the fat-soluble active ingredient is any formulation containing a fat-soluble active ingredient and a protective colloid wherein the formulation when dissolved, dispersed or diluted in/with water to a final concentration of the fat-soluble active ingredient of 10 ppm shows at a sample thickness of 1 cm an extinction of ≥ 0.15 absorbance units at the wavelength of maximum optical density or of a shoulder of optical density in the range of from 200 to 800 nm. This is equivalent to a formal extinction coefficient of the fat-soluble active ingredient in aqueous dispersion E(1%, 1 cm) of 150. The measuring of E1/1 is explicitly described in example 1.

The amount of the fat-soluble active ingredient in the formulations of the present invention may usually be from 0.1 to 90 weight-%, especially from 0.1 to 80 weight-% and from 1 to 50 weight-%. Preferably it may vary from 1 to 30 weight-%, preferably from 1 to 20 weight-%, more preferably from 1 to 15 weight-%, more preferably from 5 to 10 weight-%, based on the total weight of the composition.

Possible protective colloids encompass (modified) plant gums (preferably gum arabic, gum acacia), (cross-linked) gelatine (from any origin such as pork, beef, poultry, fish), (modified) starch, ligninsulphonate, sugar, pectins such as apple and citrus pectin, sugarbeet pectin, modified pectin such as amidated pectin, maltodextrin, lupin and plant proteins. In some embodiments of the present invention cold-water soluble protective colloids such as fish gelatine are preferred. "Cold-water soluble" in the context of the present invention means that the formulation of the protective colloid and the fat-soluble active ingredient is totally dissolved to a dispersion at a temperature from 10 to 15°C, preferably at a temperature of 10°C.

In other embodiments of the present invention the protective colloid is preferably selected from the group consisting of gelatine, modified starch, and any mixture of them. Optionally it may further contain starch, sugar and/or vegetable oil.

If a mixture of gelatine and sugar is used as protective colloid, the weight ratio of gelatine to sugar preferably varies from 20 : 1 to 1 : 10, preferably from 2 : 1 to 1 : 2.

The sugar may be selected from the group consisting of sucrose, lactose, fructose, trehalose, dextrins, maltodextrins, yellow dextrins, inverted sugars, palatinit, sorbitol, polydextrose, starch syrups, glucose syrups. Glucose syrups are e.g. commercially available from Roquette Frères (Lestrem, France) (Glucidex IT 47^{®}), from National Starch & Chemical (Bridgewater New York, USA), from Cerestar (Cargill, USA) or from Haubourdin (France).

If a mixture of modified food starch and sugar is used as the protective colloid, the weight-ratio of the modified food starch to the sugar preferably varies from 500 : 1 to 1 : 100, preferably from 20 : 1 to 1 : 10, more preferably from 10 : 1 to 1 : 10, even more preferably from 4 : 1 to 1 : 2, most preferably from 2 : 1 to 1 : 2.

In the case of mixtures of sugar, gelatine and vegetable oil as the protective colloid, the weight-ratio of the fat-soluble active ingredient to the oil preferably varies from 1 : 100 to 1 : 0, preferably from 1 : 10 to 1 : 0, more preferably from 1 : 1 to 1 : 0.

The vegetable oil may be selected from the group consisting of corn oil, soy bean oil, peanut oil, safflower oil, sunflower oil, olive oil, rapeseed oil, medium chain triglyceride oil, palm oil, palm kernel oil, cotton seed oil, and coconut oil.

The starch may be selected from the group consisting of corn starch, potato starch, tapioca starch and any mixture of them.

A preferred example of the modified (food) starch is starch modified with OSA (octenyl succinic anhydride), so called "OSA modified starch". Such modified food starches are commercially available from companies like National Starch & Chemical (Bridgewater New York, USA) (e.g. Capsul^{®}HS, Hi Cap 100^{®}, Hi Cap 200^{®}, Purity Gum 2000^{®} or Roquette Frères (Lestrem, France) (e.g. LAB 2600^{®}).

Preferred embodiments of the present invention are compositions where the fat-soluble active ingredient is either lutein, lycopene, β-carotene or zeaxanthine and the protective colloid is a mixture of modified food starch and sugar. Especially preferred are lutein forms that comprise 0.1 to 25 weight-%, preferably 1 to 20 weight-%, more preferably 2 to 10 weight-% of lutein, and/or 65 to 75 weight-% of the protective colloid (especially with a weight ratio of modified food starch to sugar of 5 : 1 to 1 : 1, preferably of 3.5 : 1 to 2.5 : 1), and/or 2 to 5 weight-% of anti-oxidants, based on the total weight of the composition. From the lycopene forms especially those are preferred that comprise 5 to 15 (preferably 10 to 14) weight-% of lycopene, and/or 55 to 75 weight-% of the protective colloid (especially with a weight ratio of modified food starch to sugar of 5 : 1 to 20 : 1, preferably of 7 : 1 to 18 : 1), and/or 2 to 5 weight-% of anti-oxidants, based on the total weight of the composition. Especially preferred are β-carotene forms that comprise 1 to 30 weight-%, preferably 5 to 30 weight-%, more preferably 15 to 30 weight-%, most preferably 15 to 25 weight-%, of β-carotene, and/or 20 to 75 weight-%, preferably 30 to 70 weight-%, more preferably 50 to 65 weight-%, of the protective colloid (especially with a weight ratio of modified food starch to sugar of 1 : 1 to 100 : 1, preferably of 1 : 1 to 70 : 1, more preferably 1 : 1 to 60 : 1), and/or 2 to 5 weight-% of anti-oxidants, based on the total weight of the composition. Especially preferred are zeaxanthin forms that comprise 1 to 20 weight-%, preferably 1 to 15 weight-%, more preferably 2 to 10 weight-% of zeaxanthin, and/or 50 to 90 weight-%, preferably 60 to 80 weight-%, more preferably 65 to 75 weight-%, of the protective colloid (especially with a weight ratio of modified food starch to sugar of 1 : 1 to 5 : 1, preferably of 1.5 : 1 to 3.5 : 1), and/or 2 to 5 weight-% of anti-oxidants, based on the total weight of the composition. More preferably these compositions are manufactured according to the process described below, whereby especially corn starch is used as powder-catch agent. Most preferably the thus resulting compositions contain from 10 to 25 weight-% of corn starch, based on the total weight of the composition.

Preferred are formulations whose preparation comprises the following steps:
a) providing a protective colloid and a fat-soluble active ingredient;
b) preparing an aqueous nano-emulsion of said protective colloid and said active ingredient, wherein the mean particle size of the particles of the inner phase of the prepared nano-emulsion is ≤ 1000 nm;
c) converting the nano-emulsion into a powder, preferably into a beadlet.

### Step b)

The nano-emulsion may be prepared by providing a protective colloid in a hydrophilic solvent such as water and adding the fat-soluble active ingredient as such or suspended/dissolved in a lipophilic solvent such as food-grade oil, chloroforme, methylene chloride, ethyl acetate, propyl acetate, hexane, heptane and/or mixtures thereof. Alternatively a lipophobic solvent lice alcohols, acetone, propanol, water and/or mixtures thereof can be used in which the fat-soluble (lipophilic) active ingredient has to be dissolved/dispersed using high temperature and pressure.

The mean particle size of the particles (Sauter diameter, D[3,2]) of the inner phase of the prepared nano-emulsion may preferably vary from 10 to 1000 nm, more preferably from 10 to 500 nm, most preferably from 200 to 300 nm, measured by laser diffraction (e.g. using the MasterSizer of Malvern, United Kingdom).

The preparation of the nano-emulsion is preferably carried out by the use of a highpressure homogenizer, a shear-blade agitator or any other suitable device known to the person skilled in the art.

### Step c)

The conversion of the nano-emulsion to a powder may be manufactured by any process known to the person skilled in the art may it be spray-drying, powder-catch or(micro)encapsulation. Preferred is the powder-catch process, especially comprising the following steps:
◇ spraying simultaneously the nano-emulsion, preferably having a temperature of from 15 to 80°C, and a powder-catch agent selected from the group consisting of starch, especially corn starch, potato starch and/or tapioca starch, calcium silicate, calcium phosphate and silicon dioxide, and a stream of hot air, preferably air having a temperature from 40 to 200°C, more preferably from 60 to 120°C, preferably through separate inlets, onto a fluidized bed of cold air, preferably air having a temperature of from 0 to 40°C, preferably from 5 to 20°C;
0 collecting the thus formed beadlets from the fluidized bed;
0 further drying the beadlets in a conventional dryer.

Beadlets in the context of the present invention are, thus, particles having an outer layer of the powder-catch agent. If the powder-catch agent is a starch it may optionally be fluidized by using silicondioxide.

A preferred embodiment of the powder-catch process is the following:
- feeding in the upper section of a vertical spray tower the nano-emulsion, preferably having a temperature of from 15 to 80°C, and, preferably through separate inlets, a powder-catch agent selected from the group consisting of starch (optionally be fluidized by using silicondioxide), especially corn starch, potato starch and/or tapioca starch, calcium silicate, calcium phosphate and silicon dioxide, and stream of hot air, preferably air having a temperature from 40 to 200°C, more preferably from 60 to 120°C;
- feeding in the lower section of said spray tower a stream of cold air, preferably air having a temperature from 0 to 40°C, preferably from 5 to 20°C, to form a fluidized bed of particles containing the fat-soluble active ingredient embedded in a matrix of the protective colloid and covered by the powder-catch agent;
- collecting said particles from the fluidized bed and
- drying them in any way known to the person skilled in the art.

The hot air used in step c) is preferably dehumidified, e.g. to a water content of less than 3 g/kg.

An alternative powder-catch process is disclosed in WO 2004/062382, p. 2,1. 12 to p. 3, 1. 35 and in the example of WO 2004/062382, whereby "the matrix component" corresponds to the protective colloid in the present invention.

The "beadlets" obtained by the powder-catch process may have a mean particle size (Sauter diameter, D[3,2]) of from 50 to 1000 µm, preferably of from 80 to 700 µm, more preferably from 100 to 500 µm, most preferably from 200 to 400 µm, as measured by laser diffraction (e.g. using the MasterSizer of Malvern, United Kingdom).

In preferred embodiments of the present invention the beadlets contain from 0.1 to 90 weight-%, preferably from 0.1 to 80 weight-%, more preferably from 1 to 50 weight-%, even more preferably from 1 to 30 weight-%, most preferably from 1 to 20 weight-%, of the fat-soluble active ingredient, from 10 to 90 weight-%, preferably from 20 to 75 weight-%, more preferably from 30 to 70 weight-%, even more preferably from 50 to 65 weight-%, of the protective colloid, from 1 to 60 weight-%, preferably from 5 to 40 weight-%, more preferably from 10 to 35 weight-%, most preferably from 15 to 30 weight-%, of the powder-catch agent and from 0.1 to 20 weight-%, preferably from 0.5 to 10 weight-%, more preferably from 1 to 5 weight-%, most preferably from 2 to 5 weight-% of antioxidants, based on the total weight of the beadlet.

Thus the present invention also refers to a formulation of a fat-soluble active ingredient with improved bioavailability comprising the formulation being prepared according to the steps a), b) and c) as described above, in comparison to a composition not being prepared according to those steps, as well as to a method for improving the bioavailability of a fat soluble ingredient in a formulation by preparing the formulation according to the steps a), b) and c) as described above.

The present invention is also directed to a process for the manufacture of a formulation of a fat-soluble active ingredient with high bioavailability of said fat-soluble active ingredient comprising the steps a), b) and c) as described above, as well as to the formulations of the fat-soluble active ingredients with high bioavailability themselves as obtainable or obtained by such a process. Such formulations contain the fat-soluble active ingredient in the form of nano-droplets/nano-particles. If such formulations are dissolved in water the nano-droplets/nano-particles are released. These nano-droplets/nano-particles have a mean particle size (Sauter diameter, D[3,2]) of the inner phase of the then formed emulsion of preferably of from 10 to 1000 nm, more preferably of from 10 to 500 nm, most preferably of from 200 to 300 nm.

A further preferred object of the present invention is an animal-free formulation containing as fat-soluble active ingredient lutein, lycopene and/or β-carotene and as protective colloid a modified food starch (with the preferences as described above) characterized in that said formulation when dissolved, dispersed or diluted in/with water has an extinction E1/1 at a wavelength of 200 to 800 nm of ≥ 380 (preferred ranges as described above) and an extrusion loss of fat-soluble active ingredient of ≥ 15% (preferred ranges as described above) when pressed to tablets. The high colour intensity of the formulation when dissolved, dispersed or diluted in/with water leads to a good release of the lutein, lycopene and/or β-carotene and, thus, to a potentially high bioavailability. The very low extrusion loss results in an excellent stability in (pharmaceutical) tablets.

Furthermore, the present invention is directed to the use of the formulations according to the present invention as dietary supplement, food, feed, personal care product, pharmaceutical with high bioavailability of said fat-soluble active ingredient.

Beside the fat-soluble active ingredient and the protective colloid the compositions of the present invention may preferably additionally contain at least one water-soluble antioxidant and/or fat-soluble antioxidant.

The water-soluble antioxidant may be for example ascorbic acid or a salt thereof, preferably sodium ascorbate, watersoluble polyphenols such as hydroxytyrosol and oleuropein aglycon, epigallocatechingallate (EGCG) or extracts of rosemary or olives.

The fat-soluble antioxidant may be for example a tocopherol, e.g. dl-α-tocopherol (i.e. synthetic tocopherol), d-α-tocopherol (i.e. natural tocopherol), β- or γ-tocopherol, or a mixture of two or more of these; butylated hydroxytoluene (BHT); butylated hydroxyanisole (BHA); ethoxyquin, propyl gallate; tert. butyl hydroxyquinoline; or 6-methoxy-1,2-dihydroxy-2,2,4-trimethylquinoline (EMQ), or an ascorbic acid ester of a fatty acid, preferably ascorbyl palmitate or stearate.

In an especially preferred embodiment of the formulation of the present invention it contains at least one antioxidant selected from the group consisting of DL-α-tocopherol, ascorbyl palmitate, sodium ascorbate, ethoxyquin and mixtures thereof.

The formulations according to the present invention may further be pressed into tablets, whereby one or more excipients and/or adjuvants selected from the group consisting of monosaccharides, disaccharides; oligosaccharides and polysaccharides, glycerol, and triglycerides, may be added.

Examples of mono- and disaccharides which may be present in the compositions of the present invention are sucrose, invert sugar, xylose, glucose, fructose, lactose, maltose, saccharose and sugar alcohols.

Examples of the oligo- and polysaccharides are starch, modified starch and starch hydrolysates, e.g. dextrins and maltodextrins, especially those having the range of 5 to 65 dextrose equivalents (DE), and glucose syrup, especially such having the range of 20 to 95 DE. The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

The triglyceride is suitably a vegetable oil or fat, preferably corn oil, sunflower oil, soybean oil, safflower oil, rapeseed oil, peanut oil, palm oil, palm kernel oil, cotton seed oil, olive oil or coconut oil.

Solid compositions may in addition contain an anti-caking agent, such as silicic acid or tricalcium phosphate and the like, and up to 10 weight-%, as a rule 2 to 5 weight-%, of water.

It was surprisingly found that the formulations of the present invention have a high bioavailability. "High bioavailability" in the context of the present invention means that the amount of fat-soluble active ingredient found in the blood plasma of the animals including humans to which it is orally applied is at least two times higher, preferably at least three times higher, than the amount of fat-soluble active ingredient being released from a formulation whose extinction E1/1 ≤ 380 at the maximum or shoulder wavelength measured by the method described in example 1, preferably from a formulation having a protective colloid from the same group as the colour-intensive formulation according to the present invention.

Animals including humans in the context of the present invention encompass besides humans especially farm animals such as sheep, cow, horses, poultry (broiler and egg pigmentation), shrimps and fish (especially salmon and rainbow trout) as well as pets such as cat, dogs, birds (e.g. flamingos) and fish.

Other aspects of the invention are food, beverages, animal feed, cosmetics and pharmaceutical compositions containing a composition as described above.

Beverages wherein the compositions of the present invention can be used, especially as a colorant or a functional ingredient, can be carbonated beverages e.g., flavoured seltzer waters, soft drinks or mineral drinks, as well as non-carbonated beverages e.g. flavoured waters, fruit juices, fruit punches and concentrated forms of these beverages. They may be based on natural fruit or vegetable juices or on artificial flavours. Also included are alcoholic beverages and instant beverage powders. Besides, sugar containing beverages, diet beverages with non-caloric and artificial sweeteners are also included.

Further, dairy products, obtained from natural sources or synthetic, are within the scope of the food products wherein the compositions of the present invention can be used, especially as a colorant or as a functional ingredient. Typical examples of such products are milk drinks, ice cream, cheese, yoghurt and the like. Milk replacing products such as soy-milk drinks and tofu products are also comprised within this range of application.

Also included are sweets which contain the compositions of the present invention as a colorant or as a functional ingredient, such as confectionery products, candies, gums, desserts, e.g. ice cream, jellies, puddings, instant pudding powders and the like.

Also included are cereals, snacks, cookies, pasta, soups and sauces, mayonnaise, salad dressings and the like which contain the compositions of the present invention as a colorant or a functional ingredient. Furthermore, fruit preparations used for dairy and cereals are also included.

The final concentration of the (fat-soluble) active ingredient and/or the colorant which is added via the compositions of the present invention to the food products may be from 0.1 to 500 ppm, particularly from 1 to 50 ppm, based on the total weight of the food composition and depending on the particular food product to be coloured or fortified and the intended grade of coloration or fortification.

The food compositions of this invention are preferably obtained by adding to a food product the (fat-soluble) active ingredient and/or the colorant in the form of a composition of this invention. For coloration or fortification of a food or a pharmaceutical product a composition of this invention can be used according to methods per se known for the application of water dispersible solid compositions of the present invention.

In general the composition may be added either as an aqueous stock solution, a dry powder mix or a pre-blend with other suitable food ingredients according to the specific application. Mixing can be done e.g. using a dry powder blender, a low shear mixer, a highpressure homogeniser or a high shear mixer depending on the formulation of the final application. As will be readily apparent such technicalities are within the skill of the expert.

Pharmaceutical compositions such as tablets or capsules wherein the compositions are used as a colorant are also within the scope of the present invention. The coloration of tablets can be accomplished by adding the compositions of the present invention in form of a liquid or solid colorant composition separately to the tablet coating mixture or by adding a colorant composition to one of the components of the tablet coating mixture. Coloured hard or soft-shell capsules can be prepared by incorporating a colorant composition in the aqueous solution of the capsule mass.

Pharmaceutical compositions such as tablets such as chewable tablets, effervescent tablets or filmcoated tablets or capsules such as hard shell capsules wherein the compositions are used as an active ingredient are also within the scope of the present invention. The compositions of the present invention are typically added as powders to the tableting mixture or filled into the capsules in a manner per se known for the production of capsules.

Animal feed products such as premixes of nutritional ingredients, compound feeds, milk replacers, liquid diets or feed preparations wherein the compositions are either used as a colorant for pigmentation e.g. for egg yolks, table poultry, broilers or aquatic animals (especially shrimps, salmon, rainbow trout) or as an active ingredient are also within the scope of the present invention.

Cosmetics, toiletries and derma products i.e. skin and hair care products such as creams, lotions, baths, lipsticks, shampoos, conditioners, sprays or gels wherein the compositions are used as a colorant or as an active ingredient are also within the scope of the present invention.

The present invention is further illustrated by the following examples.

### Examples

### Example 1

An adequate amount of the formulation is dispersed, dissolved and/or diluted in/with water by use of ultrasconics in a water bath of 50 to 55°C. The resulting "solution" is diluted to a final concentration of the fat-soluble active ingredient of 10 ppm and its UV/VIS-spectrum is measured against water as reference. From the resulting UV/VIS spectrum the absorbance at the specified wavelength of maximum or shoulder, Amax, is determined. Furthermore, the absorbance at 650 nm, A650, is determined. The color intensity E1/1 is the absorbance of a 1% solution and a thickness of 1 cm and is calculated as follows: E1/1 = (Amax-A650)*dilution factor / (weight of sample * content of product form in %).

### Examples 2 to 15

The weight-% ("wt.-%") given are based on the total weight of the formulation. "Oil" means vegetable oil.

| **Example** | **Fat-soluble active ingredient** | **Amount of fat-soluble active ingredient** | **Composition of protective colloid** | **E1/1** | **Wavelength of maximum absorption or of shoulder** | **Extrusion loss** |
|---|---|---|---|---|---|---|
| **2** | β-Carotene | 20 wt.-% | gelatine/sucrose | 900 | 416 nm (max.) | <5% |
| **3** | β-Apo-8'-carotenal | 10 wt.% | gelatine/sucrose/oil | 1300 | 460 nm (max.) | <5% |
| **4** | Lycopene | 10 wt.-% | gelatine/sucrose/oil | 400 | 480 nm (shoulder) | <5% |
| **5** | Lycopene | 5 wt.-% | gelatine/sucrose | 400 | 480 nm (shoulder) | < 5% |
| **6** | Lutein | 5 wt.-% | gelatine/sucrose | 1900 | 372 nm (shoulder) | < 5% |
| **7** | Zeaxanthin | 5 wt.-% | gelatine/sucrose | 1200 | 450 nm (max.) | <5% |
| **8** | Canthaxanthin | 10 wt.-% | gelatine/sucrose/oil | 1100 | 470 nm (max.) | < 5% |
| **9** | Canthaxanthin | 10 wt.-% | gelatine/sucrose/dextrin | 650 | 470 nm (max.) | < 5% |
| **10** | Astaxanthin | 8 wt.-% | gelatine/sucrose/dextrin | 700 | 480 nm (max.) | < 5% |
| **11** | Apocarotenoic ester | 10 wt.-% | gelatine/sucrose/dextrin | 650 | 428 nm (max.) | <5% |
| **12** | Citranaxanthin | 15 wt.-% | gelatine/sucrose/dextrin | 950 | 468 nm (max.) | < 5% |
| **13** | β-Carotene | 10 wt.-% | starch/oil | 900 | 416 nm (max.) | < 5% |
| **14** | Lycopene | 10 wt.-% | modified food starch/glucose syrup | 450 | 480 nm (shoulder) | <5% |
| **15** | Lutein | 5 wt.-% | modified food starch/glucose syrup | 1500 | 372 nm (shoulder) | <5% |

The compositions mentioned in the table additionally contain at least one antioxidant selected from the group consisting of DL-α-tocopherol, ascorbyl palmitate, sodium ascorbate, ethoxyquin and mixtures thereof.

### Comparison Examples 16 to 18

These formulations are not prepared according to the processes described above.

| **Example** | **Fat-soluble active ingredient** | **Amount of fat-soluble active ingredient** | **protective colloid** | **E1/1** | **Wavelength of maximum absorption or of shoulder** | **Extrusion loss** |
|---|---|---|---|---|---|---|
| **16** | lutein esters | 9.5% | gelatine, sucrose, palm oil | 350 | 372 nm (shoulder) | 49% |
| **17** | lutein | 15% | sucrose monolaurate | 220 | 372 nm (shoulder) | 100% |
| **18** | lutein | 5% | sucrose, tapioca starch | 100 | 372 nm (shoulder) | 67% |

### Example 19: Bioavailability studies with β-carotene - comparison of the bioavailability of formulations according to the present invention and those of the prior art

### General design of the studies

On days -7 and 0, a blood sample was collected from each volunteer for determination of baseline serum β-carotene. Immediately following the collection of the second baseline blood sample (day 0), each volunteer was given the day's supplement of β-carotene (15 mg). Following this, the volunteers were given a breakfast.

From day 0 until day 14 of the study, each volunteer repeated the β-car lowed by breakfast. In addition, on days 2, 4, 7, 10 and 14, blood samples were collected. The changes from baseline serum β-carotene values were used to compare bioavailability data of different formulations. The area under the curve is used as relative bioavailability indicator, and is expressed in % of a defined standard formulation (= the formulation with the higher bioavailability).

| **Product** | **β-carotene - prior art formulation** | **β-carotene Beta-Tab®** |
|---|---|---|
| **Content of β-carotene (measured by UV)** | 22.0% | 21.5 wt.-% |
| **E1/1** | 374 | 721 |
| **protective colloid** | gelatine | gelatine/sucrose |
| **Bioavailability measured as blood plasma level** | 43% | 100% |

## Claims

1. A formulation of a fat-soluble active ingredient with high bioavailability comprising a fat-soluble active ingredient and a protective colloid **characterized in that** said formulation when dissolved, dispersed or diluted in/with water has an extinction E1/1 at a wavelength in the range of from 200 to 800 nm of ≥ 380,
**characterized in that** the fat-soluble active ingredient is selected from the group consisting of carotenoids and fat-soluble vitamins, as well as their physiologically acceptable esters and any mixtures of them; and
**characterized in that** the carotenoid is selected from the group consisting of β-carotene, 8'-apo-β-carotenal, lutein, zeaxanthin, lycopene, astaxantin, canthaxanthin, citranaxanthin, 8'-apo-β-carotenoic acid ethyl ester and any mixture of them,
and **characterized in that** the protective colloid is selected from the group consisting of plant gums, modified plant gums, gelatine, cross-linked gelatine, starch, modified starch, ligninsulphonate, sugar, apple pectin, citrus pectin, modified pectin, maltodextrin and plant proteins.

2. The formulation as claimed in claim 1, **characterized in that** the formulation shows an extrusion loss of fat-soluble active ingredient of ≤ 30% when pressed to tablets.

3. The formulation as claimed in claim 1 and/or 2, **characterized in that** the process for the manufacture of said formulation comprises the following steps:
a) providing a protective colloid and a fat-soluble active ingredient;
b) preparing an aqueous nano-emulsion of said protective colloid and said active ingredient, wherein the mean particle size of the particles of the inner phase of the prepared nano-emulsion is ≤ 1000 nm;
c) converting the nano-emulsion into a powder, preferably into a beadlet.

4. The formulation as claimed in claim 3, **characterized in that** step c is carried out by a powder-catch process.

5. The formulation as claimed in any one of the preceding claims, **characterized in that** said formulation when dissolved, dispersed or diluted in/with water has an extinction E1/1 at a wavelength in the range of from 200 to 800 nm ≥ 600.

6. The formulation as claimed in any one of the preceding claims, **characterized in that** said formulation when dissolved, dispersed or diluted in/with water has an extinction E1/1 at a wavelength in the range of from 250 to 600 nm ≥ 380.

7. The formulation as claimed in any one of the preceding claims, **characterized in that** said formulation when dissolved, dispersed or diluted in/with water has an extinction E1/1 at a wavelength in the range of from 250 to 600 nm ≥ 600.

8. The formulation as claimed in any one of the preceding claims, **characterized in that** said formulation when dissolved, dispersed or diluted in/with water has an extinction E1/1 at a wavelength in the range of from 370 to 485 nm ≥ 380, more preferably ≥ 600, most preferably ≥ 900 nm.

9. The formulation as claimed in any one of the preceding claims, **characterized in that** the protective colloid is selected from the group consisting of gelatine, starch, sugar, vegetable oil and any mixture of them.

10. The formulation as claimed in any one of the preceding claims, **characterized in that** the protective colloid is selected from the group consisting of gelatine, modified starch, and any mixture of them.

11. The formulation as claimed in claim 10 **characterized in that** the protective colloid further contains starch, sugar and / or vegetable oil.

12. The formulation as claimed in any one of the preceding claims **characterized in that** the carotenoid is selected from the group consisting of β-carotene, lutein, lycopene, astaxanthin, canthaxanthin and zeaxanthin.

13. The formulation as claimed in any one of the preceding claims, **characterized in that** it contains from 0.1 to 90 weight-%, preferably from 1 to 80 weight-%, more preferably from 1 to 50 weight-%, even more preferably from 1 to 30 weight-%, most preferably from 1 to 20 weight-%, of a fat-soluble active ingredient, and from 10 to 90 weight-%, preferably from 20 to 75 weight-%, more preferably from 30 to 70 weight-%, of a protective colloid, based on the total weight of the formulation.

14. The formulation as claimed in claim 13, **characterized in that** it additionally contains from 1 to 60 weight-%, preferably from 5 to 40 weight-%, more preferably from 10 to 35 weight-%, most preferably from 15 to 30 weight-%, of a powder-catch agent and from 0.1 to 20 weight-%, preferably from 0.5 to 10 weight-%, more preferably from 1 to 5 weight-% of antioxidants, based on the total weight of the formulation.

15. The formulation as claimed in any one of the preceding claims, **characterized in that** the fat-soluble active ingredient is lutein, lycopene, β-carotene or zeaxanthin and that the protective colloid is a mixture of modified food starch and sugar.

16. The formulation as claimed in claim 15, **characterized in that** it contains 0.1 to 25 weight-%, preferably 1, to 20 weight-%, more preferably 2 to 10 weight-%, of lutein, and/or 65 to 75 weight-% of the protective colloid (especially with a weight ratio of modified food starch to sugar of 1 : 1 to 5 : 1, preferably of 2.5 : 1 to 3.5 : 1), and/or 2 to 5 weight-% of anti-oxidants and/or 10 to 25 weight-% of corn starch, based on the total weight of the formulation.

17. The formulation as claimed in claim 15, **characterized in that** it contains 5 to 15 (preferably 10 to 14) weight-% of lycopene, and/or 55 to 75 weight-% of the protective colloid (especially with a weight ratio of modified food starch to sugar of 5 : 1 to 20 : 1, preferably of 7 ; 1 to 18 : 1), and/or 2 to 5 weight-% of anti-oxidants and/or 10 to 25 weight-% of corn starch, based on the total weight of the composition.

18. The formulation as claimed in claim 15, **characterized in that** it contains 1 to 30 weight-%, preferably 5 to 30 weight-%, more preferably 15 to 30 weight-%, most preferably 15 to 25 weight-%, of β-carotene, and/or 20 to 75 weight-%, preferably 30 to 70 weight-%, more preferably 50 to 65 weight-%, of the protective colloid (especially with a weight ratio of modified food starch to sugar of 1 : 1 to 100 : 1, preferably of 1 : 1 to 70 : 1, more preferably 1 : 1 to 60 : 1), and/or 2 to 5 weight-% of anti-oxidants and/or 10 to 25 weight-% of corn starch, based on the total weight of the composition.

19. The formulation as claimed in claim 15, **characterized in that** it contains from 1 to 20 (preferably 1 to 15) weight-% of zeaxanthin, and/or 50 to 90 weight-%, preferably 60 to 80 weight-%, more preferably 65 to 75 weight-%, of the protective colloid (especially with a weight ratio of modified food starch to sugar of 1 : 1 to 5 : 1, preferably of 1.5 : 1 to 3.5 : 1), and/or 2 to 5 weight-% of anti-oxidants and/or 10 to 25 weight-% of corn starch, based on the total weight of the composition.

20. An animal-free formulation containing as fat-soluble active ingredient lutein, lycopene and/or β-carotene and as protective colloid a modified food starch **characterized in that** said formulation when dissolved, dispersed or diluted in/with water has an extinction E1/1 at a wavelength, in the range of from 200 to 800 nm of ≥ 380 and an extrusion loss of fat-soluble active ingredient of ≤ 15% when pressed to tablets.

21. A formulation of a fat-soluble active ingredient with improved bioavailability comprising the formulation being prepared according to the following steps a), b) and c):
a) providing a protective colloid and a fat-soluble active ingredient;
b) preparing an aqueous nano-emulsion of said protective colloid and said active ingredient, wherein the mean particle size of the particles of the inner phase of the prepared nano-emulsion is ≤ 1000 mm;
c) converting the nano-emulsion into a powder, preferably by a powder-catch process;
in comparison to a formulation not being prepared according to these steps, **characterized in that** the fat-soluble active ingredient is selected from the group consisting of carotenoids and fat-soluble vitamins, as well as their physiologically acceptable esters and any mixtures of them; and
**characterized in that** the carotenoid is selected from the group consisting of β-carotene, 8'-apo-β-carotenal, lutein, zeaxanthin, lycopene, astaxantin, canthaxanthin, citranaxanthin, 8'-apo-β-carotenoic acid ethyl ester and any mixture of them, and **characterized in that** the protective colloid is selected from the group consisting of plant gums, modified plant gums, gelatine, cross-linked gelatine, starch, modified starch, ligninsulphonate, sugar, apple pectin, citrus pectin, modified pectin, maltodextrin and plant proteins.

22. A process for the manufacture of a formulation of a fat-soluble active ingredient with high bioavailability of said fat-soluble active ingredient comprising the following steps:
a) providing a protective colloid and a fat-soluble active ingredient;
b) preparing an aqueous nano-emulsion of said protective colloid and said active ingredient, wherein the mean particle size of the particles of the inner phase of the prepared nano-emulsion is ≤ 1000 nm;
c) converting the nano-emulsion into a powder, preferably by a powder-catch process, **characterized in that** the fat-soluble active ingredient is selected from the group consisting of carotenoids and fat-soluble vitamins, as well as their physiologically acceptable esters and any mixtures of them; and
**characterized in that** the carotenoid is selected from the group consisting of β-carotene, 8'-apo-β-carotenal, lutein, zeaxanthin, lycopene, astaxantin, canthaxanthin, citranaxanthin, 8'-apo-β-carotenoic acid ethyl ester and any mixture of them, and
**characterized in that** the protective colloid is selected from the group consisting of plant gums, modified plant gums, gelatine, cross-linked gelatine, starch, modified starch, ligninsulphonate, sugar, apple pectin, citrus pectin, modified pectin, maltodextrin and plant proteins.

23. Formulations of a fat-soluble active ingredient with high bioavailability obtainable by the process of claim 22.

24. Use of a formulation as claimed in claims 1 to 21 and 23 as dietary supplement, food, feed, personal care product, pharmaceutical with high bioavailability of said fat-soluble active ingredient.

25. A method for improving the bioavailability of a fat soluble ingredient in a formulation by preparing the formulation according to the following steps:
a) providing a protective colloid and a fat-soluble active ingredient;
b) preparing an aqueous nano-emulsion of said protective colloid and said active ingredient, wherein the mean particle size of the particles of the inner phase of the prepared nano-emulsion is ≤ 1000 nm;
c) converting the nano-emulsion into a powder, preferably by a powder-catch process,
**characterized in that** the fat-soluble active ingredient is selected from the group consisting of carotenoids and fat-soluble vitamins, as well as their physiologically acceptable esters and any mixtures of them; and
**characterized in that** the carotenoid is selected from the group consisting of β-carotene, 8'-apo-β-carotenal, lutein, zeaxanthin, lycopene, astaxantin, canthaxanthin, citranaxanthin, 8'-apo-β-carotenoic acid ethyl ester and any mixture of them, and
**characterized in that** the protective colloid is selected from the group consisting of plant gums, modified plant gums, gelatine, cross-linked gelatine, starch, modified starch, lignin-sulphonate, sugar, apple pectin, citrus pectin, modified pectin, maltodextrin and plant proteins.

## Patentansprüche

1. Formulierung eines fettlöslichen Wirkstoffs mit hoher Bioverfügbarkeit, umfassend einen fettlöslichen Wirkstoff und ein Schutzkolloid, **dadurch gekennzeichnet, dass** die Formulierung, wenn in/mit Wasser gelöst, dispergiert oder verdünnt, eine Extinktion E1/1 bei einer Wellenlänge im Bereich von 200 bis 800 nm von ≥ 380 aufweist,
**dadurch gekennzeichnet, dass** der fettlösliche Wirkstoff ausgewählt ist aus der Gruppe bestehend aus Carotenoiden und fettlöslichen Vitaminen sowie deren physiologisch annehmbaren Estern und beliebigen Gemischen davon; und
**dadurch gekennzeichnet, dass** das Carotenoid ausgewählt ist aus der Gruppe bestehend aus β-Caroten, 8'-Apo-β-carotenal, Lutein, Zeaxanthin, Lycopen, Astaxanthin, Canthaxanthin, Citraxanthin, 8'-Apo-β-carotensäureetylester und beliebigen Gemischen davon,
und **dadurch gekennzeichnet, dass** das Schutzkolloid ausgewählt ist aus der Gruppe bestehend aus Pflanzengummis, modifizierten Pflanzengummis, Gelatine, vernetzter Gelatine, Stärke, modifizierter Stärke, Ligninsulfonat, Zucker, Apfel-Pectin, Citrus-Pectin, modifiziertem Pectin, Maltodextrin und Pflanzenproteinen.

2. Formulierung gemäß Anspruch 1, **dadurch gekennzeichnet**, das die Formulierung, wenn zu Tabletten gepresst, einen Extrusionsverlust an fettlöslichem Wirkstoff von ≤ 30 % aufweist.

3. Formulierung gemäß Anspruch 1 und/oder 2, **dadurch gekennzeichnet, dass** das Verfahren zum Herstellen der Formulierung folgende Schritte umfasst:
a) Bereitstellen eines Schutzkolloids und eines fettlöslichen Wirkstoffs;
b) Herstellen einer wässrigen Nanoemulsion des Schutzkolloids und des Wirkstoffs, wobei die mittlere Partikelgröße der Partikel der inneren Phase der hergestellten Nanoemulsion ≤ 1000 nm beträgt;
c) Umwandeln der Nanoemulsion in ein Pulver, vorzugsweise zu Kügelchen.

4. Formulierung gemäß Anspruch 3, **dadurch gekennzeichnet, dass** Schritt c durch ein Pulverfangverfahren durchgeführt wird.

5. Formulierung gemäß einem der vorstehenden Ansprüche, **dadurch gekennzeichnet, dass** die Formulierung, wenn in/mit Wasser gelöst, dispergiert oder verdünnt, eine Extinktion E1/1 bei einer Wellenlänge im Bereich von 200 bis 800 nm von ≥ 600 aufweist.

6. Formulierung gemäß einem der vorstehenden Ansprüche, **dadurch gekennzeichnet, dass** die Formulierung, wenn in/mit Wasser gelöst, dispergiert oder verdünnt, eine Extinktion E1/1 bei einer Wellenlänge im Bereich von 250 bis 600 nm von ≥ 380 aufweist.

7. Formulierung gemäß einem der vorstehenden Ansprüche, **dadurch gekennzeichnet, dass** die Formulierung, wenn in/mit Wasser gelöst, dispergiert oder verdünnt, eine Extinktion E1/1 bei einer Wellenlänge im Bereich von 250 bis 600 nm von ≥ 600 aufweist.

8. Formulierung gemäß einem der vorstehenden Ansprüche, **dadurch gekennzeichnet, dass** die Formulierung, wenn in/mit Wasser gelöst, dispergiert oder verdünnt, eine Extinktion E1/1 bei einer Wellenlänge im Bereich von 370 bis 485 nm von ≥ 380 aufweist, bevorzugter ≥ 600, höchst bevorzugt ≥ 900.

9. Formulierung gemäß einem der vorstehenden Ansprüche, **dadurch gekennzeichnet, dass** das Schutzkolloid ausgewählt ist aus der Gruppe bestehend aus Gelatine, Stärke, Zucker, Pflanzenöl und beliebigen Gemischen davon.

10. Formulierung gemäß einem der vorstehenden Ansprüche, **dadurch gekennzeichnet, dass** das Schutzkolloid ausgewählt ist aus der Gruppe bestehend aus Gelatine, modifizierter Stärke und beliebigen Gemischen davon.

11. Formulierung gemäß Anspruch 10, **dadurch gekennzeichnet, dass** das Schutzkolloid ferner Stärke, Zucker und/oder Pflanzenöl enthält.

12. Formulierung gemäß einem der vorstehenden Ansprüche, **dadurch gekennzeichnet, dass** das Caroteinoid ausgewählt ist aus der Gruppe bestehend aus β-Caroten, Lutein, Lycopen, Astaxanthin, Canthaxanthin und Zeaxanthin.

13. Formulierung gemäß einem der vorstehenden Ansprüche, **dadurch gekennzeichnet, dass** sie von 0,1 bis 90 Gew.-%, vorzugsweise von 1 bis 80 Gew.-%, bevorzugter von 1 bis 50 Gew.-%, noch bevorzugter von 1 bis 30 Gew.-%, höchst bevorzugt von 1 bis 20 Gew.-% an einem fettlöslichen Wirkstoff und von 10 bis 90 Gew.-%, vorzugsweise von 20 bis 75 Gew.-%, bevorzugter von 30 bis 70 Gew.-% an einem Schutzkolloid, bezogen auf das Gesamtgewicht der Formulierung, enthält.

14. Formulierung gemäß Anspruch 13, **dadurch gekennzeichnet, dass** sie zusätzlich von 1 bis 60 Gew.-%, vorzugsweise von 5 bis 40 Gew.-%, bevorzugter von 10 bis 35 Gew.-%, höchst bevorzugt von 15 bis 30 Gew.-% an einem pulverfangenden Mittel und von 0,1 bis 20 Gew.-%, vorzugsweise von 0,5 bis 10 Gew.-%, bevorzugter von 1 bis 5 Gew.-% an Antioxidationsmitteln, bezogen auf das Gesamtgewicht der Formulierung, enthält.

15. Formulierung gemäß einem der vorstehenden Ansprüche, **dadurch gekennzeichnet, dass** der fettlösliche Wirkstoff Lutein, Lycopen, β-Caroten oder Zeaxanthin ist und dass das Schutzkolloid ein Gemisch von modifizierter Lebensmittelstärke und Zucker ist.

16. Formulierung gemäß Anspruch 15, **dadurch gekennzeichnet, dass** sie 0,1 bis 25 Gew.-%, vorzugsweise 1 bis 20 Gew.-%, bevorzugter 2 bis 10 Gew.-% an Lutein und/oder 65 bis 75 Gew.-% an dem Schutzkolloid (insbesondere mit einem Gewichtsverhältnis von modifizierter Lebensmittelstärke zu Zucker von 1 : 1 bis 5 : 1, vorzugsweise von 2,5 : 1 bis 3,5 : 1) und/oder 2 bis 5 Gew.-% an Antioxidationsmitteln und/oder 10 bis 25 Gew.-% an Maisstärke, bezogen auf das Gesamtgewicht der Formulierung, enthält.

17. Formulierung gemäß Anspruch 15, **dadurch gekennzeichnet, dass** sie von 5 bis 15 (vorzugsweise 10 bis 14) Gew.-% an Lycopen und/oder 55 bis 75 Gew.-% an dem Schutzkolloid (insbesondere mit einem Gewichtsverhältnis von modifizierter Lebensmittelstärke zu Zucker von 5 : 1 bis 20 : 1, vorzugsweise von 7 : 1 bis 18 : 1) und/oder 2 bis 5 Gew.-% an Antioxidationsmitteln und/oder 10 bis 25 Gew.-% an Maisstärke, bezogen auf das Gesamtgewicht der Formulierung, enthält.

18. Formulierung gemäß Anspruch 15, **dadurch gekennzeichnet, dass** sie 1 bis 30 Gew.-%, vorzugsweise 5 bis 30 Gew.-%, bevorzugter 15 bis 30 Gew.-%, höchst bevorzugt 15 bis 25 Gew.-% an β-Caroten und/oder 20 bis 75 Gew.-%, vorzugsweise 30 bis 70 Gew.-%, bevorzugter 50 bis 65 Gew.-% an dem Schutzkolloid (insbesondere mit einem Gewichtsverhältnis von modifizierter Lebensmittelstärke zu Zucker von 1 : 1 bis 100 : 1, vorzugsweise von 1 : 1 bis 70 : 1, bevorzugter von 1 : 1 bis 60 : 1) und/oder 2 bis 5 Gew.-% an Antioxidationsmitteln und/oder 10 bis 25 Gew.-% an Maisstärke, bezogen auf das Gesamtgewicht der Formulierung, enthält.

19. Formulierung gemäß Anspruch 15, **dadurch gekennzeichnet, dass** sie von 1 bis 20 (vorzugsweise 1 bis 15) Gew.-% an Zeaxanthin und/oder 50 bis 90 Gew.-%, vorzugsweise 60 bis 80 Gew.-%, bevorzugter 65 bis 75 Gew.-% an dem Schutzkolloid (insbesondere mit einem Gewichtsverhältnis von modifizierter Lebensmittelstärke zu Zucker von 1 : 1 bis 5 : 1, vorzugsweise von 1,5 : 1 bis 3,5 : 1) und/oder 2 bis 5 Gew.-% an Antioxidationsmitteln und/oder 10 bis 25 Gew.-% an Maisstärke, bezogen auf das Gesamtgewicht der Formulierung, enthält.

20. Tierfreie Formulierung, die als fettlöslichen Wirkstoff Lutein, Lycopen und/oder β-Caroten und als Schutzkolloid eine modifizierte Lebensmittelstärke enthält, **dadurch gekennzeichnet, dass** die Formulierung, wenn in/mit Wasser gelöst, dispergiert oder verdünnt, eine Extinktion E1/1 bei einer Wellenlänge im Bereich von 200 bis 800 nm von ≥ 380 und, wenn zu Tabletten gepresst, einen Extrusionsverlust an fettlöslichem Wirkstoff von ≤ 15 % aufweist.

21. Formulierung eines fettlöslichen Wirkstoffs mit verbesserter Bioverfügbarkeit, umfassend die gemäß den folgenden Schritten a), b) und c) hergestellte Formulierung:
a) Bereitstellen eines Schutzkolloids und eines fettlöslichen Wirkstoffs;
b) Herstellen einer wässrigen Nanoemulsion des Schutzkolloids und des Wirkstoffs, wobei die mittlere Partikelgröße der Partikel der inneren Phase der hergestellten Nanoemulsion ≤ 1000 nm beträgt;
c) Umwandeln der Nanoemulsion in ein Pulver, vorzugsweise durch ein Pulverfangverfahren;
im Vergleich zu einer Formulierung, die nicht gemäß diesen Schritten hergestellt ist,
**dadurch gekennzeichnet, dass** der fettlösliche Wirkstoff ausgewählt ist aus der Gruppe bestehend aus Carotenoiden und fettlöslichen Vitaminen sowie deren physiologisch annehmbaren Estern und beliebigen Gemischen davon; und
**dadurch gekennzeichnet, dass** das Carotenoid ausgewählt ist aus der Gruppe bestehend aus β-Caroten, 8'-Apo-β-carotenal, Lutein, Zeaxanthin, Lycopen, Astaxanthin, Canthaxanthin, Citraxanthin, 8'-Apo-β-carotensäureetylester und beliebigen Gemischen davon, und
**dadurch gekennzeichnet, dass** das Schutzkolloid ausgewählt ist aus der Gruppe bestehend aus Pflanzengummis, modifizierten Pflanzengummis, Gelatine, vernetzter Gelatine, Stärke, modifizierter Stärke, Ligninsulfonat, Zucker, Apfel-Pectin, Citrus-Pectin, modifiziertem Pectin, Maltodextrin und Pflanzenproteinen.

22. Verfahren zum Herstellen einer Formulierung eines fettlöslichen Wirkstoffs mit hoher Bioverfügbarkeit des fettlöslichen Wirkstoffs, umfassend folgende Schritte:
a) Bereitstellen eines Schutzkolloids und eines fettlöslichen Wirkstoffs;
b) Herstellen einer wässrigen Nanoemulsion des Schutzkolloids und des Wirkstoffs, wobei die mittlere Partikelgröße der Partikel der inneren Phase der hergestellten Nanoemulsion ≤ 1000 nm beträgt;
c) Umwandeln der Nanoemulsion in ein Pulver, vorzugsweise durch ein Pulverfangverfahren;
**dadurch gekennzeichnet, dass** der fettlösliche Wirkstoff ausgewählt ist aus der Gruppe bestehend aus Carotenoiden und fettlöslichen Vitaminen sowie deren physiologisch annehmbaren Estern und beliebigen Gemischen davon; und
**dadurch gekennzeichnet, dass** das Carotenoid ausgewählt ist aus der Gruppe bestehend aus β-Caroten, 8'-Apo-β-carotenal, Lutein, Zeaxanthin, Lycopen, Astaxanthin, Canthaxanthin, Citraxanthin, 8'-Apo-β-carotensäureetylester und beliebigen Gemischen davon, und
**dadurch gekennzeichnet, dass** das Schutzkolloid ausgewählt ist aus der Gruppe bestehend aus Pflanzengummis, modifizierten Pflanzengummis, Gelatine, vernetzter Gelatine, Stärke, modifizierter Stärke, Ligninsulfonat, Zucker, Apfel-Pectin, Citrus-Pectin, modifiziertem Pectin, Maltodextrin und Pflanzenproteinen.

23. Formulierungen eines fettlöslichen Wirkstoffs mit hoher Bioverfügbarkeit, erhältlich durch das Verfahren gemäß Anspruch 22.

24. Verwendung einer Formulierung gemäß Ansprüchen 1 bis 21 und 23 als Nahrungsergänzungsmittel, Lebensmittel, Futtermittel, Körperpflegeprodukt, Arzneimittel mit hoher Bioverfügbarkeit des fettlöslichen Wirkstoffs.

25. Verfahren zum Verbessern der Bioverfügbarkeit eines fettlöslichen Inhaltsstoffs in einer Formulierung durch Herstellen der Formulierung gemäß folgenden Schritten:
a) Bereitstellen eines Schutzkolloids und eines fettlöslichen Wirkstoffs;
b) Herstellen einer wässrigen Nanoemulsion des Schutzkolloids und des Wirkstoffs, wobei die mittlere Partikelgröße der Partikel der inneren Phase der hergestellten Nanoemulsion ≤ 1000 nm beträgt;
c) Umwandeln der Nanoemulsion in ein Pulver, vorzugsweise durch ein Pulverfangverfahren;
**dadurch gekennzeichnet, dass** der fettlösliche Wirkstoff ausgewählt ist aus der Gruppe bestehend aus Carotenoiden und fettlöslichen Vitaminen sowie deren physiologisch annehmbaren Estern und beliebigen Gemischen davon; und
**dadurch gekennzeichnet, dass** das Carotenoid ausgewählt ist aus der Gruppe bestehend aus β-Caroten, 8'-Apo-β-carotenal, Lutein, Zeaxanthin, Lycopen, Astaxanthin, Canthaxanthin, Citraxanthin, 8'-Apo-β-carotensäureetylester und beliebigen Gemischen davon, und
**dadurch gekennzeichnet, dass** das Schutzkolloid ausgewählt ist aus der Gruppe bestehend aus Pflanzengummis, modifizierten Pflanzengummis, Gelatine, vernetzter Gelatine, Stärke, modifizierter Stärke, Ligninsulfonat, Zucker, Apfel-Pectin, Citrus-Pectin, modifiziertem Pectin, Maltodextrin und Pflanzenproteinen.

## Revendications

1. Formulation d'une substance active liposoluble ayant une biodisponibilité élevée comprenant une substance active liposoluble et un colloïde protecteur, **caractérisée en ce que** ladite formulation, lorsqu'elle est dissoute, dispersée ou diluée dans/avec de l'eau a une extinction E1/1 à une longueur d'onde dans la plage de 200 à 800 nm ≥ 380,
**caractérisée en ce que** la substance active liposoluble est choisie dans le groupe constitué de caroténoïdes et de vitamines liposolubles, ainsi que leurs esters physiologiquement acceptables et des mélanges quelconques de ceux-ci ; et
**caractérisée en ce que** le caroténoïde est choisi dans le groupe constitué des β-carotène, 8'-apo-β-caroténal, lutéine, zéaxanthine, lycopène, astaxantine, canthaxanthine, citranaxanthine, ester éthylique d'acide 8'-apo-β-caroténoïque et un mélange quelconque de ceux-ci,
et **caractérisée en ce que** le colloïde protecteur est choisi dans le groupe constitué de gommes végétales, gommes végétales modifiées, gélatine, gélatine réticulée, amidon, amidon modifié, ligninesulfonate, sucre, pectine de pomme, pectine d'agrume, pectine modifiée, maltodextrine et protéines végétales.

2. Formulation selon la revendication 1, **caractérisée en ce que** la formulation présente une perte à l'extrusion de substance active liposoluble ≤ 30 % lorsqu'elle est pressée en comprimés.

3. Formulation selon la revendication 1 et/ou 2, **caractérisée en ce que** le procédé de fabrication de ladite formulation comprend les étapes suivantes :
a) fourniture d'un colloïde protecteur et d'une substance active liposoluble ;
b) préparation d'une nano-émulsion aqueuse dudit colloïde protecteur et de ladite substance active, la taille de particule moyenne des particules de la phase interne de la nano-émulsion préparée étant ≤ 1000 nm ;
c) conversion de la nano-émulsion en poudre, de préférence en granules.

4. Formulation selon la revendication 3, **caractérisée en ce que** l'étape c est conduite par un procédé de capture de poudre.

5. Formulation selon l'une quelconque des revendications précédentes, **caractérisée en ce que** ladite formulation, lorsqu'elle est dissoute, dispersée ou diluée dans/avec de l'eau a une extinction E1/1 à une longueur d'onde dans la plage de 200 à 800 nm ≤ 600.

6. Formulation selon l'une quelconque des revendications précédentes, **caractérisée en ce que** ladite formulation, lorsqu'elle est dissoute, dispersée ou diluée dans/avec de l'eau a une extinction E1/1 à une longueur d'onde dans la plage de 250 à 600 nm ≤ 380.

7. Formulation selon l'une quelconque des revendications précédentes, **caractérisée en ce que** ladite formulation, lorsqu'elle est dissoute, dispersée ou diluée dans/avec de l'eau a une extinction E1/1 à une longueur d'onde dans la plage de 250 à 600 nm ≤ 600.

8. Formulation selon l'une quelconque des revendications précédentes, **caractérisée en ce que** ladite formulation, lorsqu'elle est dissoute, dispersée ou diluée dans/avec de l'eau a une extinction E1/1 à une longueur d'onde dans la plage de 370 à 485 nm ≥ 380, plus préférablement ≥ 600, de manière préférée entre toutes ≥ 900 nm.

9. Formulation selon l'une quelconque des revendications précédentes, **caractérisée en ce que** le colloïde protecteur est choisi dans le groupe constitué de gélatine, amidon, sucre, huile végétale et un mélange quelconque de ceux-ci.

10. Formulation selon l'une quelconque des revendications précédentes, **caractérisée en ce que** le colloïde protecteur est choisi dans le groupe constitué de gélatine, amidon modifié, et un mélange quelconque de ceux-ci.

11. Formulation selon la revendication 10 **caractérisée en ce que** le colloïde protecteur contient en outre de l'amidon, du sucre et/ou de l'huile végétale.

12. Formulation selon l'une quelconque des revendications précédentes **caractérisée en ce que** le caroténoïde est choisi dans le groupe constitué des β-carotène, lutéine, lycopène, astaxanthine, canthaxanthine et zéaxanthine.

13. Formulation selon l'une quelconque des revendications précédentes, **caractérisée en ce qu'**elle contient de 0,1 à 90 % en poids, de préférence de 1 à 80 % en poids, plus préférablement de 1 à 50 % en poids, encore plus préférablement de 1 à 30 % en poids, de manière préférée entre toutes de 1 à 20 % en poids, d'une substance active liposoluble, et de 10 à 90 % en poids, de préférence de 20 à 75 % en poids, plus préférablement de 30 à 70 % en poids, d'un colloïde protecteur, sur la base du poids total de la formulation.

14. Formulation selon la revendication 13, **caractérisée en ce qu'**elle contient en outre de 1 à 60 % en poids, de préférence de 5 to 40 % en poids, plus préférablement de 10 à 35 % en poids, de manière préférée entre toutes de 15 à 30 % en poids, d'un agent de capture de poudre et de 0,1 à 20 % en poids, de préférence de 0,5 à 10 % en poids, plus préférablement de 1 à 5 % en poids d'antioxydants, sur la base du poids total de la formulation.

15. Formulation selon l'une quelconque des revendications précédentes, **caractérisée en ce que** la substance active liposoluble est la lutéine, le lycopène, le β-carotène ou la zéaxanthine et que le colloïde protecteur est un mélange d'amidon alimentaire modifié et de sucre.

16. Formulation selon la revendication 15, **caractérisée en ce qu'**elle contient de 0,1 à 25 % en poids, de préférence de 1 à 20 % en poids, plus préférablement de 2 à 10 % en poids, de lutéine, et/ou de 65 à 75 % en poids du colloïde protecteur (en particulier avec un rapport en poids de l'amidon alimentaire modifié au sucre de 1 : 1 à 5 : 1, de préférence de 2,5 : 1 à 3,5 : 1), et/ou de 2 à 5 % en poids d'antioxydants et/ou de 10 à 25 % en poids d'amidon de maïs, sur la base du poids total de la formulation.

17. Formulation selon la revendication 15, **caractérisée en ce qu'**elle contient de 5 à 15 (de préférence de 10 à 14) % en poids de lycopène, et/ou de 55 à 75 % en poids du colloïde protecteur (en particulier avec un rapport en poids de l'amidon alimentaire modifié au sucre de 5 : 1 à 20 : 1, de préférence de 7 : 1 à 18 : 1), et/ou de 2 à 5 % en poids d'antioxydants et/ou de 10 à 25 % en poids d'amidon de maïs, sur la base du poids total de la composition.

18. Formulation selon la revendication 15, **caractérisée en ce qu'**elle contient de 1 à 30 % en poids, de préférence de 5 à 30 % en poids, plus préférablement de 15 à 30 % en poids, de manière préférée entre toutes de 15 à 25 % en poids, de β-carotène, et/ou de 20 à 75 % en poids, de préférence de 30 à 70 % en poids, plus préférablement de 50 à 65 % en poids, du colloïde protecteur (en particulier avec un rapport en poids de l'amidon alimentaire modifié au sucre de 1 : 1 à 100 : 1, de préférence de 1 : 1 à 70 : 1, plus préférablement de 1 : 1 à 60 : 1), et/ou de 2 à 5 % en poids d'antioxydants et/ou de 10 à 25 % en poids d'amidon de maïs, sur la base du poids total de la composition.

19. Formulation selon la revendication 15, **caractérisée en ce qu'**elle contient de 1 à 20 (de préférence de 1 à 15) % en poids de zéaxanthine, et/ou de 50 à 90 % en poids, de préférence de 60 à 80 % en poids, plus préférablement de 65 à 75 % en poids, du colloïde protecteur (en particulier avec un rapport en poids de l'amidon alimentaire modifié au sucre de 1 : 1 à 5 : 1, de préférence de 1,5 : 1 à 3,5 : 1), et/ou de 2 à 5 % en poids d'antioxydants et/ou de 10 à 25 % en poids d'amidon de maïs, sur la base du poids total de la composition.

20. Formulation sans animal contenant, en tant que substance active liposoluble, de la lutéine, du lycopène et/ou du β-carotène et en tant que colloïde protecteur, un amidon alimentaire modifié, **caractérisée en ce que** ladite formulation, lorsqu'elle est dissoute, dispersée ou diluée dans/avec de l'eau a une extinction E1/1 à une longueur d'onde dans la plage de 200 à 800 nm ≥ 380 et une perte à l'extrusion de substance active liposoluble ≤ 15 % lorsqu'elle est pressée en comprimés.

21. Formulation d'une substance active liposoluble ayant une biodisponibilité améliorée comprenant la formulation étant préparée selon les étapes suivantes a), b) et c) :
a) fourniture d'un colloïde protecteur et d'une substance active liposoluble ;
b) préparation d'une nano-émulsion aqueuse dudit colloïde protecteur et de ladite substance active, la taille de particule moyenne des particules de la phase interne de la nano-émulsion préparée étant ≤ 1000 nm ;
c) conversion de la nano-émulsion en poudre, de préférence par un procédé de capture de poudre ;
par rapport à une formulation n'étant pas préparée selon ces étapes,
**caractérisée en ce que** la substance active liposoluble est choisie dans le groupe constitué de caroténoïdes et de vitamines liposolubles, ainsi que leurs esters physiologiquement acceptables et des mélanges quelconques de ceux-ci ; et
**caractérisée en ce que** le caroténoïde est choisi dans le groupe constitué des β-carotène, 8'-apo-β-caroténal, lutéine, zéaxanthine, lycopène, astaxantine, canthaxanthine, citranaxanthine, ester éthylique d'acide 8'-apo-β-caroténoïque et un mélange quelconque de ceux-ci, et
**caractérisée en ce que** le colloïde protecteur est choisi dans le groupe constitué de gommes végétales, gommes végétales modifiées, gélatine, gélatine réticulée, amidon, amidon modifié, ligninesulfonate, sucre, pectine de pomme, pectine d'agrume, pectine modifiée, maltodextrine et protéines végétales.

22. Procédé de fabrication d'une formulation d'une substance active liposoluble ayant une biodisponibilité élevée de ladite substance active liposoluble comprenant les étapes suivantes :
a) fourniture d'un colloïde protecteur et d'une substance active liposoluble ;
b) préparation d'une nano-émulsion aqueuse dudit colloïde protecteur et de ladite substance active, la taille de particule moyenne des particules de la phase interne de la nano-émulsion préparée étant ≤ 1000 nm ;
c) conversion de la nano-émulsion en poudre, de préférence par un procédé de capture de poudre, **caractérisé en ce que** la substance active liposoluble est choisie dans le groupe constitué de caroténoïdes et de vitamines liposolubles, ainsi que leurs esters physiologiquement acceptables et des mélanges quelconques de ceux-ci ; et
**caractérisé en ce que** le caroténoïde est choisi dans le groupe constitué des β-carotène, 8'-apo-β-caroténal, lutéine, zéaxanthine, lycopène, astaxantine, canthaxanthine, citranaxanthine, ester éthylique d'acide 8'-apo-β-caroténoïque et un mélange quelconque de ceux-ci, et
**caractérisé en ce que** le colloïde protecteur est choisi dans le groupe constitué de gommes végétales, gommes végétales modifiées, gélatine, gélatine réticulée, amidon, amidon modifié, ligninesulfonate, sucre, pectine de pomme, pectine d'agrume, pectine modifiée, maltodextrine et protéines végétales.

23. Formulations d'une substance active liposoluble ayant une biodisponibilité élevée pouvant être obtenues par le procédé de la revendication 22.

24. Utilisation d'une formulation selon les revendications 1 à 21 et 23 en tant que supplément alimentaire, aliment pour humains, aliment pour animaux, produit de soin personnel, agent pharmaceutique ayant une biodisponibilité élevée de ladite substance active liposoluble.

25. Procédé d'amélioration de la biodisponibilité d'une substance liposoluble dans une formulation par préparation de la formulation selon les étapes suivantes :
a) fourniture d'un colloïde protecteur et d'une substance active liposoluble ;
b) préparation d'une nano-émulsion aqueuse dudit colloïde protecteur et de ladite substance active, la taille de particule moyenne des particules de la phase interne de la nano-émulsion préparée étant ≤ 1000 nm ;
c) conversion de la nano-émulsion en poudre, de préférence par un procédé de capture de poudre, **caractérisé en ce que** la substance active liposoluble est choisie dans le groupe constitué de caroténoïdes et de vitamines liposolubles, ainsi que leurs esters physiologiquement acceptables et des mélanges quelconques de ceux-ci ; et
**caractérisé en ce que** le caroténoïde est choisi dans le groupe constitué des β-carotène, 8'-apo-β-caroténal, lutéine, zéaxanthine, lycopène, astaxantine, canthaxanthine, citranaxanthine, ester éthylique d'acide 8'-apo-β-caroténoïque et un mélange quelconque de ceux-ci, et
**caractérisé en ce que** le colloïde protecteur est choisi dans le groupe constitué de gommes végétales, gommes végétales modifiées, gélatine, gélatine réticulée, amidon, amidon modifié, ligninesulfonate, sucre, pectine de pomme, pectine d'agrume, pectine modifiée, maltodextrine et protéines végétales.
